**Europäisches Patentamt**

**European Patent Office**

**Office européen des brevets**

(11) Veröffentlichungsnummer: **0 073 436**
**A2**

## (12) EUROPÄISCHE PATENTANMELDUNG

(21) Anmeldenummer: **82107652.8**

(51) Int. Cl.³: **C 12 N 15/00**

(22) Anmeldetag: **20.08.82**

(30) Priorität: **24.08.81 JP 132328/81**

(71) Anmelder: **MEIJI SEIKA KABUSHIKI KAISHA, 4-16 Kyobashi 2-chome, Chuo-ku, Tokyo-To (JP)**

(72) Erfinder: **Nojiri, Chuhei, 4275-28, Izumi-Cho Totsuka-Ku, Yokohama-Chi Kanagawa-Ken (JP)**
Erfinder: **Katsumata, Kazuko, 165-11 Kariba-Cho Hydogaya-Ku, Yokohama-Shi Kanagawa-Ken (JP)**
Erfinder: **Yamada, Yujiro, 385-13, Shimoda-Cho Kouhoku-Ku, Yokohama-Shi Kanagawa-Ken (JP)**

(43) Veröffentlichungstag der Anmeldung: **09.03.83**
**Patentblatt 83/10**

(74) Vertreter: **Dr. Elisabeth Jung Dr. Jürgen Schirdewahn Dr. Gerhard Schmitt-Nilson Dr. Gerhard B. Hagen Dipl.-Ing. Peter Hirsch, Clemensstrasse 30 Postfach 40 14 68, D-8000 München 40 (DE)**

(84) Benannte Vertragsstaaten: **CH DE FR GB LI NL**

(54) **Hybridplasmid und dessen Herstellung.**

(57) A hybrid plasmid comprising of a plurality of unit plasmids joined together is disclosed, which hybrid plasmid is proliferable in a Gram-positive bacterium and is stabilized against dissociation thereof into the parent unit plasmids by the removal of the base sequence related to the dissociation. A process for the preparation is also disclosed.

EP 0 073 436 A2

# 0073436

## HYBRID PLASMID AND PREPARATION THEREOF

### BACKGROUND OF THE INVENTION

This invention relates to a novel plasmid. More particularly, the present invention pertains to a hybrid plasmid comprising a plurality of plasmids joined together. The present invention also concerns a process for producing the hybrid plasmid.

Various kinds of hybrid plasmids comprising a plurality of plasmids joined together are already known in the art, and in fact most of the newly constructed plasmids may be said to be hybrid plasmids.

Plasmids, which are useful as vectors for cloning of foreign DNA in cells of bacteria and for transformation of bacteria, are now becoming of interest in researches of host-vector systems for Gram-positive bacteria such as Bacillus subtilis as host bacteria in place of Gram-negative bacteria such as E. coli. Some hybrid plasmids have been proposed for Bacillus subtilis, although fewer than those for E. coli. For example, there are reported pTA 1245 from pTP4 and pTP5 (Agric. Biol. Chem 44 (1), 2601 (1980)), pBD12 from pUB110 and pC194 (Proc. Natl. Acad. Sci. U.S.A. 75 (3), 1428 (1978)), and pHV 11 from pC194 and pT127 (Proc. Natl. Acad. Sci. U.S.A. 75 (3), 1433 (1978)). Each of these hybrid plasmids has been formed by treating different plasmids having the same restriction endonuclease cleavage sites with the same restriction endonuclease and joining them together.

### SUMMARY OF THE INVENTION

The present invention is intended for providing a hybrid plasmid for Gram-positive bacteria such as Bacillus subtilis, which has been obtained according to a novel process and is based on the discovery of the formation of a joined body of plural species of plasmids joined together within cells of Bacillus subtilis and also on the recognition of the problem involved in the joined plasmids as well as improvement thereof.

Thus, the hybrid plasmid according to the present invention is a joined body of plural species of unit plasmids, characterized in that the base sequence related to dissociation into the unit plasmids has been removed from its structure and also that it is proliferable in a Gram-positive bacterium.

The process for producing the hybrid plasmid according to the present invention comprises introducing plural species of unit plasmids proliferable in Bacillus subtilis cells into Bacillus subtilis cells, culturing these cells to form a joined body of the unit plasmids within the cells, recovering the joined body of plasmids, treating the joined body of plasmids with a restriction endonuclease to excise the base sequence related to dissociation into the unit plasmids in the joined body of plasmids and then ligating again the cleaved fragments with each other.

A new plasmid for Gram-positive bacteria and a new process for production of the plasmid are provided by the present invention, and the plasmid, namely the hybrid plasmid, can be obtained to be conveniently used as a vector by suitable selection of unit plasmids. For example, by selection of unit plasmids each having at least one kind of selection markers which are designed so that they will be present at the sites which are not removed during excision of the dissociation-related base sequence or the restriction endonuclease cleavage site may be present within the gene to be utilized as the selection marker to form "inactivation by insertion" (such a design can readily be feasible and the hybrid plasmid of the present invention is most commonly so designed), the hybrid plasmid formed can be easily recovered (as described in detail below), and the choice of transformed strains by the hybrid plasmid can easily be made. It is also easy to design a hybrid plasmid so that it will have a small molecular weight of, for example, 6 megadalton (Md) or less, and therefore the hybrid plasmid can be obtained with a size suitable for its utilization as a vector.

As mentioned above, the present invention is based on the discovery of in vivo joining of plasmids. In addition, with a recognition of the problem that the thus formed joined plasmids are relatively unstable and not suitable for use as a vector, the problem has been solved by excising the base sequence related to dissociation of the hybrid plasmid into unit plasmids. It appears that there has been no report on in vivo formation of joined plasmids. The joined body of plasmids, while it is relatively unstable, can be made stable by elimination of the based sequence related to dissociation to unit plasmids, whereby cloning of different kinds of DNA can be stably performed.

It should also be noted that the hybrid plasmid according to the present invention is within the "Guidelines for recombinant DNA researches" in Japan, so far as safety is concerned.

BRIEF DESCRIPTION OF THE DRAWINGS

In the drawings:

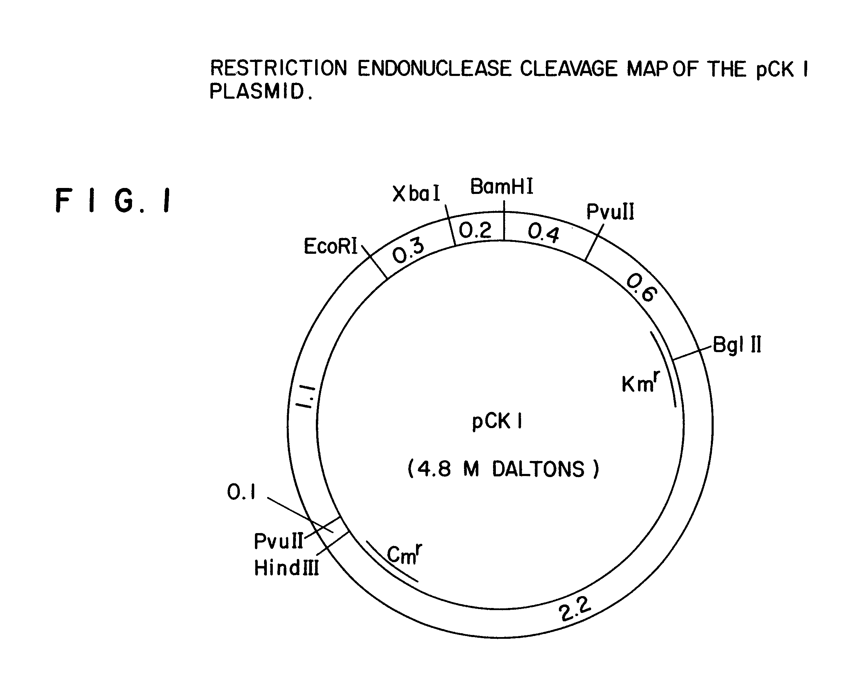

FIGS. 1, 2, and 3 are restriction endonuclease cleavage maps respectively of the pCK 1 and the hybrid plasmids pCK 2 and pCK 3 according to the present invention.

DETAILED DESCRIPTION OF THE INVENTION

1. Hybrid plasmid:

1) Contents

The hybrid plasmid comprises plural kinds of unit plasmids, especially those proliferable in Bacillus subtilis cells, joined together.

According to a preferred embodiment of the present invention, at least one kind of unit plasmids is selected from the group consisting of pUB 110 and pE 194. As a usual practice, two kinds of unit plasmids are joined together. The plural kinds of unit plasmids to be combined are also required to be compatible with each other at the place where joining thereof is to be carried out, namely, within microorganism cells, above all, within Bacillus subtilis cells.

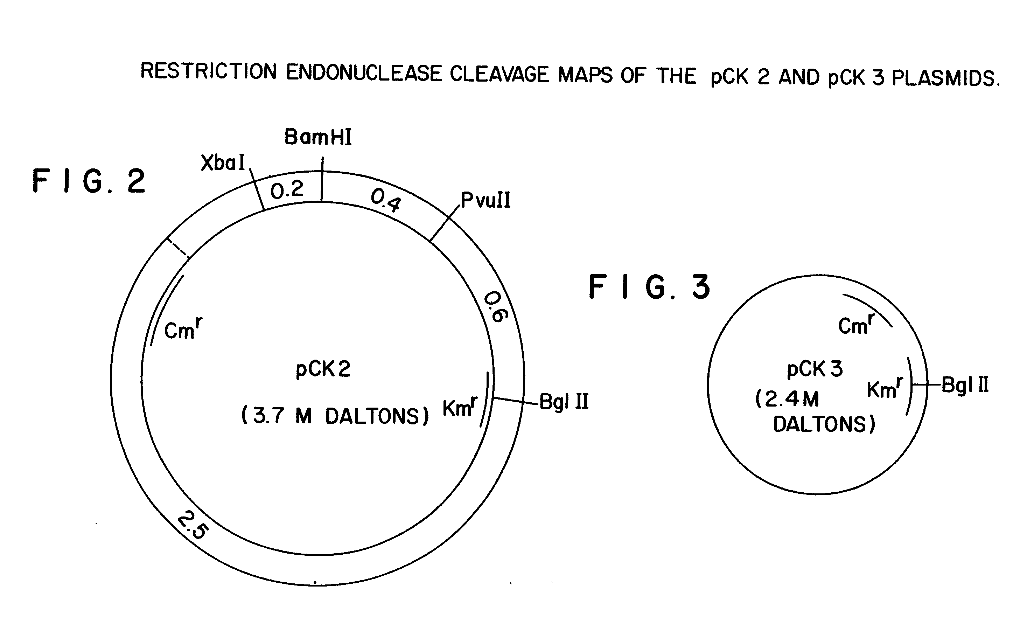

Examples of joinable unit plasmid combinations are those of pUB 110 (Km$^r$) with pC194 (Cm$^r$), pTP4 (Cm$^r$), pTP5 (Tc$^r$ (tetracycline resistant)), pT127 (Tc$^r$) and pE194 (Em$^r$ (erythromycin resistant)); and those of pE194 (Em$^r$) with pC194 (Cm$^r$), pTP4 (Cm$^r$), pTP5 (Tc$^r$) and pT127 (Tc$^r$). Representative of these examples is the combination of pUB 110 and pC 194, which can provide a preferred example of the present invention with the finished hybrid plasmid after excision of the base sequence related to dissociation, viz. pCK 2 (M.W.= 3.7 Md) and pCK 3 (M.W.= 2.4 Md). Each of these plasmids has two kinds of selection markers, namely Km$^r$ (kanamycin resistant) and Cm$^r$ (chloramphenicol resistant). Restriction endonuclease cleavage maps of pCK 2 and pCK 3 are as shown in FIG. 2 and FIG. 3, respectively.

The hybrid plasmid according to the present invention is proliferable in the cells of Gram-positive bacteria such as Bacillus subtilis.

2) Utility

The hybrid plasmid according to the present invention, as a plasmid for Gram-positive bacteria, especially for Bacillus subtilis, is useful as vectors for cloning of various genes and for transformation of Gram-positive bacteria.

2. Preparation of hybrid plasmid:

The hybrid plasmid according to the present invention may be prepared by a process comprising the steps of (a) joining of unit plasmids, namely, preparing a precursor of the hybrid plasmid of the present invention in microorganism cells and (b) stabilizing the precursor, namely, excising from the precursor the base sequence related to dissociation of the joined plasmid into unit plasmids.

1) In vivo joining of unit plasmids

Plural kinds of unit plasmids (as exemplified above) are introduced into the microorganism cells, which are in turn cultivated to form joined bodies of unit plasmids. As the host microorganisms to be employed in this case, Gram-positive bacteria, especially Bacillus subtilis, are preferred.

Typical examples of Bacillus subtilis suitable for this purpose are Bacillus subtilis Y12S ("FERM-BP No. 167" on deposit at the Fermentation Research Institute, Agency of Industrial Science and Technology) FERM (genotype: arg15, leuA8, non A1, non B1) and B. subtilis PS9 (releasable strains stored at Saito's Laboratory, Institute of Applied Microbiology, Tokyo University) genotype: pur B6, leuA8, thr 5, met B5, his A1, lys 21, trp C2, non A1, non B1). These are mutants of B. subtilis Marburg 168-strain which are strains deficient in restriction endonucleases. Generally speaking, all strains are available which are mutants derived from B. subtilis 168-strain and are deficient in the restriction endonuclease concerned with the unit plasmid to be introduced.

Introduction of unit plasmids into microorganism cells and cultivation of the cells may be conducted according to conventional methods. For example, plasmids may be introduced into the cells of Bacillus subtilis by introducing unit plasmids into competent cells cultured on the Spizizen's minimum culture medium, or by reacting the plasmids in the presence of polyethylene glycol with protoplasts formed by treatment of the cells with lysozyme. Plural kinds of plasmids may be introduced into the cells either at once or by stepwise additions.

Recovery of joined bodies of unit plasmids from the cells should be conducted also with due consideration of separation from the unjoined unit plasmids. For this purpose, a preferred process comprises the steps of:

(a) isolating the strains according to the selection markers possessed by the unit plasmids from the cultured product of cells, and isolating the plasmids in a conventional manner from these strains;

(b) treating the isolated plasmids with a restriction endonuclease specific to one of the unit plasmids to cleave that unit plasmid and the joined plasmid having that unit plasmid into a linear DNA, respectively;

(c)  isolating the linear DNAs from the circular DNA not cleaved which is namely the other unit plasmid;

(d)  converting the resultant linear DNA again to circular DNA by, for example, treatment with T4 ligase;

(e)  introducing the resultant DNA into Bacillus subtilis cells thereby to transform the Bacillus subtilis; and

(f)  selecting the desired transformed strains through plural selection markers of the joined plasmids and isolating the joined plasmids from the transformed strains.

A typical example of the thus prepared joined plasmids is pCK 1 (M.W.= 4.8 Md) which is the joined body of pUB 110 and pCl94.  Its restriction endonuclease cleavage map is as shown in FIG. 1.

The B. subtilis Y12S into which pCK 1 has been introduced, namely B. subtilis Y12S (pCK 1) is on deposit at the "FERM" as "FERM-BP No. 168".

2)  Stabilization of the joined plasmids

The joined plasmids constructed as described above in cells can be isolated, but they are instable and tend to be dissociated into the parent unit plasmids in the cells when they are to be used as vectors.

Therefore, it is required that the joined plasmids be stabilized according to the present invention.

Stabilization treatment of the joined plasmids comprises excising the base sequence related to dissociation into the unit plasmids comprised in the structure of the joined body.

It is not at present clear as to of what base sequence the code related to dissociation into the unit plasmids in the joined plasmids is constituted and also at which site it is positioned.  However, in the present invention, it is postulated that the base sequence around the points corresponding to the contact points of the unit plasmids, namely, the two sites in the case of a joined body of two kinds of unit plasmids, is the base sequence related to dissociation. Since the base sequence related to dissociation is interpreted as the contact point between unit plasmids, the base sequence may also be the association sites to be recognized by the host cells or the unit plasmids.

For excising the specific base sequence from the joined plasmids, an *in vitro* treatment comprising cleavage by a restriction endonuclease and joining by a ligase may be employed. Since the base sequence is understood to exist at two positions as a minimum as described above, the wording in the present invention "the base sequence related to dissociation has been removed" or "after the base sequence related to dissociation is excised" means that at least one of them is removed or excised.

The kind of the restriction endonuclease to be used is determined according to the base sequence to be excised. More specifically, for example, when the joined body of plasmids is pCK 1, the base sequence to be excised lies between <u>Eco</u> RI site - <u>Hind</u> III site or between <u>Hind</u> III site - <u>Bgl</u> II site. However, the base sequence to be excised may desirably be at the portion where no selection marker exists between <u>Eco</u> RI site - <u>Hind</u> III site in case of pCK 1.

Cleavage or ring opening by a restriction endonuclease and subsequent ligation or ring closure may be conducted according to conventional procedures. More specifically, for example, in the case of pCK 1, <u>Eco</u> RI and <u>Hind</u> III are permitted to act thereon to remove the base sequence concerned which is excised and provide a ring-opened plasmid having the <u>Eco</u> RI terminal and the <u>Hind</u> III terminal, which plasmid is then treated with a nuclease (e.g. S1, BAL - 31) to produce blunt terminals, which step is followed by ligation with the $T_4$ ligase.

8

0073436
When both of the terminals of the linear DNA after excision of the base sequence concerned are complementary to
each other, it is, of course, possible to effect ligation
directly with the $T_4$ ligase or others.

It is also within the scope of the present invention
to impart a novel restriction endonuclease cleavage site
to the desired hybrid plasmid. In such a case, during ring
closure of the above ring-opened plasmid, ligation with
the $T_4$ ligase may be accomplished with addition of various
restriction endonuclease linkers such as Eco RI, Bam HI,
Pst I, etc.

The desired hybrid plasmid can be obtained from the
thus prepared ring-closed product in a conventional manner.
The ring-closed product may thus be introduced into
Bacillus subtilis according to the protoplast method, etc.,
to cause transformation thereof, the transformed strains
being selected according to the selection markers borne
by the hybrid plasmid ($Km^r$ or $Cm^r$ in the case of pCK 2 and
pCK 3) and the desired hybrid plasmid isolated from the
selected strains.

3. Experimental examples:

Experiment 1 (in vivo preparation of hybrid plasmid
pCK 1)

Competent cells of B. subtilis Y12S having plasmid
pUB 110 (3.0 Md, $Km^r$) introduced therein were prepared by
the method of Dubnau et al [1], and 1 µg of the pC194
plasmid DNA (1.8 Md, $Cm^r$) was added to 0.5 ml of the competent cells. Incubation was carried out at 37 °C for
30 minutes to cause transformation of the cells and 0.1
ml of the transformed product was spread over a Penassay
broth agar plate containing 10 µg/ml of Cm. Cultivation
was conducted at 37 °C for 16 hours to obtain transformed
strains of $10^3$/µg DNA. All of the transformed strains
of $Cm^r$ were found to be $Km^r$.

The $Cm^r$, $Km^r$ strains were inoculated into Penassay
broth medium, and shaking cultivation was carried out at
37 °C for 16 hours and followed by centrifugation, to obtain 2.5 g of wet cells. The plasmid was separated from the cells according to the method of Leblanc, Lee [2] to obtain about 150 μg of plasmid DNA.

The DNA was divided into aliquots each of half quantity, which were digested with (A) 75 units of the restriction endonuclease <u>Hind</u> III and (B) 75 units of the restriction endonuclease <u>Eco</u> RI, respectively at 37 °C for 3 hours. Each of the digested DNA was then subjected to ethidium bromide-cesium chloride density gradient centrifugation (20 °C/6 hours using a Beckman Vertical Rotor VTi 80, Type L8-80 Ultra-centrifuge). The linear DNA fractions of (A) and (B) separated by this procedure were each thoroughly dialyzed against a buffer (20 mM Tris HCl, $MgCl_2$ 10 mM, pH 7.6), and then dithiothreitol and adenosine-5'-triphosphate (ATP) were added to the final concentrations of 10 mM and 0.6 mM, respectively. Further, with addition of 0.1 unit of the $T_4$ ligase, treatment was carried out at 22 °C for 2 hours.

Using each 5 μg of the $T_4$ ligase-treated DNA of (A) and (B), <u>B. subtilis</u> Y12S strains were transformed according to the protoplast method of Chang, Cohen et al [4] to obtain transformed strains of $Km^r$, $Cm^r$. Two strains of the $Km^r$, $Cm^r$ transformant from (A) and one strain from (B) were subjected to liquid culture, which was followed by separation of the plasmids.

Each of the plasmids was cleaved with a restriction endonuclease such as <u>Bgl</u> II, <u>Hind</u> III, <u>Bam</u> HI, <u>Xba</u> I and <u>Eco</u> RI either alone or simultaneously with two or more species, and the respective DNA fragments were analyzed by agarose gel electrophoresis to prepare a restriction endonuclease map.

All of the plasmids of three strains were found to be the same hybrid plasmid of pUB 110 and pC194 with the same directional property, and this hybrid plasmid was named pCK 1.

Experiment 2 (dissociation of pCK 1)

With the use of the pCK 1 plasmid, <u>B. subtilis</u> Y12S strain

was transformed according to the aforesaid protoplast method, and the transformed strains were spread over protoplast regeneration medium DM3[5] containing (A) 150 µg/ml of kanamycin and (B) 10 µg/ml of chloramphenicol. The transformed strains which appeared were replicated onto Penassay broth agar media, containing 10 µg/ml of Cm for (A) and 10 µg/ml of Km for (B). As a result, a strain was formed in each transformed strain of (A) and (B) with a frequency of 0.5 to 5% which became suceptible to one of the reagents.

From each of the strains which had lost one of the resistances, the plasmid was separated, cleaved with the restriction endonuclease and examined according to agrose gel electrophoresis, whereby it was found that every plasmid of $Km^r$, $Cm^s$ strains was pUB 110 and that of $Cm^r$ $Km^s$ was pCl94.

As is apparent from the above results, a part of the pCK 1 hybrid plasmid is dissociated in B. subtilis cells into the parent pUB 110 and pCl94, and such a dissociation occurs at specific sites, namely between Hind III site - Eco RI site and between Hind III site - Bgl II site in pCK 1, these sites being considered to be the same as those at which pUB 110 and pCl94 are associated to be joined together. It seems likely that the sites are similar to transposon or IS as clarified in Gram-negative bacteria.

Experiment 3 (Conversion of pCK 1 into stable plasmides pCK 2 and pCK 3)

Ten (10) µg of pCK 1 was cleaved at the same time with 10 units each of Eco RI and Hind III and subjected to 5 - 20% sucrose linear density gradient centrifugation (Beckman L8-80 Ultracentrifuge 1SW 40 rotor, 4 °C/5 hours) and the small fragments formed (1.2 Md of Eco RI - Hind III) were removed. A solution (100 µl) containing 3 µg of the principal fraction DNA was dialyzed in a buffer (0.03 M sodium citrate, 0.3 M NaCl, 4.5 mM $ZnCl_2$, pH 4.6) for 5 hours, and 3 units of S1 nuclease were added to carry out the digestion at 37 °C for one hour. Then, the reaction was stopped by addition of 20 µl of a solution of 2.5 M Tris HCL, 0.2 M EDTA, pH 8.0.

The reaction product was dialyzed in the aforesaid reaction mixture for the $T_4$ ligase and treated with addition of dithiothreitol, ATP and one unit of the $T_4$ ligase at 22 °C for 2 hours. With the use of the whole amount of the resultant mixture, B. subtilis Y12S strains were transformed according to the protoplast method.

Plasmids were separated from five strains of the transformants ($Km^r$, $Cm^r$) and analyzed according to restriction endonuclease agarose gel electrophoresis. As a result, the plasmids of four strains were found to be pCK 2, while the other one was pCK 3 which was shorter than the pCK 2 by 1.4 Md for some reason.

Experiment 4 (preparation of a hybrid plasmid (shuttle vector) of pCK 2 and an actinomycete plasmid pSF 588)

(A) After 3 µg of pCK 2 DNA was cleaved with 3 units of Bam HI at 37 °C for 2 hours, one unit of alkali phosphatase was added to cause digestion at 60 °C for one hour. The digested mixture was shaken with an equal amount of phenol saturated with a buffer A (0.1 M Tris. HCl, 20 mM EDTA, pH 8.0) and then subjected to centrifugation at 4,000 rpm for 10 minutes. The upper layer was taken out and dialyzed against a buffer B (10 mM Tris EDTA, pH 7.6).

(B) Ten (10) µg of the pSF588 plasmid DNA were treated with 10 units of Bgl II at 37 °C for 2 hours, subjected to the heat treatment at 65 °C for 10 minutes and dialyzed against the buffer B.

To each of the DNA solutions A and B, 10 mM of $MgCl_2$, 10 mM of dithiothreitol and 0.6 mM of ATP (each denoting the final cencentration) were added. The solution A was maintained at 22 °C with 0.1 unit of the $T_4$ ligase incorporated therein, and the solution B was added thereto in three divided portions at the intervals of 15 minutes. This step was followed further by incubation at 22 °C for one hour, to prepare a recombinant DNA.

B. subtilis pS9 was transformed according to the protoplast method with 50 μl of the resultant recombinant DNA solution (about 4 μg of DNA), and the plasmids were separated for examination from ten transformed strains of $Cm^r$, $Km^r$. Of the ten strains, seven plasmids were found to be the hybrid plasmid of pCK 2 and pSF 588.

Experiment 5 (cloning of an actinomycete gene with the use of pCK 2 as vector (Shot-gun method) )

(A) Cleavage of 5 μg of pCK 2 DNA was effected by digestion with 5 units of Bgl II, and, after the resultant product was treated with 2 units of alkali phosphatase at 60 °C for one hour, the phenol treatment and dialysis were carried out similarly as in Experiment 4.

(B) The chromosome DNA of the actinomycete S. griseus was extracted and purified according to the SDS-phenol method, 25 μg of the DNA obtained were cleaved with 10 units of Bgl II at 37°C for one hour and then subjected to heat treatment at 65 °C for 10 minutes. The DNA solution was dialyzed against the buffer B.

To each of the DNA solutions of (A) and (B), $MgCl_2$, dithiothreitol and ATP were added. The solution (A) was mixed with 0.2 unit of the $T_4$ ligase and, while the solution (A) was maintained at 22 °C, the solution (B) was added in four divided portions at the intervals of 15 minutes. After the treatment for an additional one hour, a recombinant DNA was obtained.

Nine strains of B. subtilis pS were transformed with 50 μl (6 μg DNA) of the recombinant DNA solution to obtain $10^4$ strains of transformed strains of $Cm^r$.

When 200 of the transformed strains were examined for Km resistance, about 80% were found to be $Km^s$ (namely, cloned strains in which "inactivation by insertion" occurred as a result of incorporation of S. griseus DNA).

Plasmids were separated from 12 strains of the $Cm^r$, $Km^s$ strains, and each after cleavage with Bgl II was subjected to analysis by agarose electrophoresis. As the result, pCK 2 and S. griseus DNA fragments in which pCK 2 had been inserted were detected in each plasmid.

4.   Deposition of microorganisms:

The Bacillus subtilis Yl2S used for in vivo joining of unit plasmids is on deposit dated August 20, 1981 at the FERMENTATION RESEARCH INSTITUTE, AGENCY OF INDUSTRIAL SCIENCE AND TECHNOLOGY, 1-3, Higashi 1-chome Yatabe-machi, Tsukuba-gun, Ibaraki-ken, Japan as "FERM P-No. 6110" and FERM BP-No. 167 under the BUDAPEST TREATY ON THE INTERNATIONAL RECOGNITION OF THE DEPOSIT OF MICROORGANISM FOR THE PURPOSES OF PATENT PROCEDURE.

The Bacillus subtilis YS12S (pCK 1), in which the in vivo joined body from pUB 110 and pC194, namely, pCK 1, is incorporated, is on deposit dated August 20, 1981 at the same depository as above as "FERM P-No. 6111" and FERM BP-No. 168 under the same BUDAPEST TREATY.

5.   References:

(1)   J. Mol. Biol. 56, 209 - 221 (1971)

(2)   J. Bact. 140, 1112 - 1115 (1979)

(3)   Proc. Natl. Acad. Sci., USA 57, 1514 - 1531 (1967)

(4)   Molec. Gen. Genet. 168, 111 - 115 (1979)

(5)   J. Bact. 116, 456 - 465 (1973)

WHAT IS CLAIMED IS:

1.     A hybrid plasmid comprising a joined body of plural species of unit plasmids, characterized in that the base sequence related to dissociation into said unit plasmids has been removed from its structure and also that it is proliferable in a Gram-positive bacterium.

2.     A hybrid plasmid according to claim 1, wherein at least one species of the unit plasmids is selected from the group consisting of pUB 110 and pE 194.

3.     A hybrid plasmid according to claim 1 or claim 2, wherein the base sequence related to dissociation into unit plasmids includes the base sequence at the contact point between the unit plasmids to be hybridized.

4.     A hybrid plasmid according to claim 1, which is pCK 2 having a molecular weight of 3.7 Md and two selection markers $Km^r$ and $Cm^r$.

5.     A hybrid plasmid according to claim 1, which is pCK 3 having a molecular weight of 2.4 Md and two selection markers $Km^r$ and $Cm^r$.

6.     A process for producing a hybrid plasmid, which comprises introducing plural species of unit plasmids proliferable in Bacillus subtilis cells into Bacillus subtilis cells, culturing said cells to form a joined body of said unit plasmids within said cells, recovering the joined body of plasmids, treating the joined body of plasmids with a restriction endonuclease to excise the base sequence related to dissociation into said unit plasmids in said joined body of plasmids and then ligating the cleaved fragments with each other.

7.     A process for producing a hybrid plasmid according to claim 6, wherein recovery of the joined body of plasmids from the cultured cells is carried out according to the procedure comprising the steps of:

    (a) isolating the plasmids from the strains, which strains are selected from the cultured cells according to the selection markers possessed by the unit plasmids;

    (b)   treating the isolated plasmids with a restriction endonuclease specific to one of the unit plasmids to cleave said unit plasmid and the joined plasmid having said unit plasmid into a linear DNA, respectively;

    (c)   isolating the linear DNAs from the circular DNA not cleaved which is the other unit plasmid;

    (d)   converting the resultant linear DNA again to circular DNA;

    (e)   introducing the resultant DNA into Bacillus subtilis cells thereby to transform the Bacillus subtilis; and

    (f)   selecting the desired transformed strains according to plural selection markers of the joined plasmids and isolating the joined plasmids from the transformed strains.

8.     A process for producing a hybrid plasmid according to claim 6 or claim 7, wherein the unit plasmids are pUB 110 ($Km^r$) and pC194 ($Cm^r$), and the base sequence related to dissociation into unit plasmids is excised by the treatment thereof with Eco RI and Hind III thereby to remove the portion between the recognition sites of said enzymes.

RESTRICTION ENDONUCLEASE CLEAVAGE MAP OF THE pCK I
PLASMID.

# F I G. I

RESTRICTION ENDONUCLEASE CLEAVAGE MAPS OF THE pCK 2 AND pCK 3 PLASMIDS.

FIG. 2

FIG. 3